# EUROPEAN PATENT APPLICATION

(11) **EP 2 962 669 A1**
(43) Date of publication of application: **06.01.2016**
(21) Application number: 14002224.5
(22) Date of filing: 30.06.2014
(51) Int. Cl.: A61F 5/48

(54) **Bed pad with one or more releasable and fastenable wings**

(71) Applicant: The Procter & Gamble Company, Cincinnati, OH 45202 (US)
(72) Inventor: Miralles, Jose, 08170 Montornès del Vallès (Barcelona) (ES); Esquerra, Juan, 08170 Montornès del Vallès (Barcelona) (ES)
(74) Representative: Heide, Ute

(57) **Abstract**

A bed pad (10) is provided and comprises an absorbent pad (11) having a first and second lateral edge (12, 13). The absorbent pad comprises a liquid permeable topsheet (14) having a first and second lateral edge, a liquid impermeable backsheet (15) and an absorbent layer (28) which is located between the topsheet and the backsheet. The bed pad further comprises at least one wing (40). The one or more wings have a first and second lateral edge (41, 43). One or more fasteners (42, 44, 45) are disposed at or adjacent to each of the first and second lateral edges of the absorbent pad and/or at or adjacent to one or both of the first and second lateral edges of the one or more wings, such that the one or more wings are able to be releasably engaged with the absorbent pad at or adjacent to the first and/or second lateral edge of the absorbent pad.

## Description

### FIELD OF THE INVENTION

The invention provides a bed pad suitable for use over bedding beneath an incontinent person. The bed pad comprises and absorbent pad and at least one wing. The one or more wings have one or more fasteners which are disposed at or adjacent to one or both of a first and second lateral edges of the one or more wings. The one or more wings can be releasably engaged with the absorbent pad at or adjacent to a first and/or second lateral edge of the absorbent pad. Moreover, the invention provides a package comprising one or more absorbent pads and one or more wings able to releasably engage with each absorbent pad of the package.

### BACKGROUND OF THE INVENTION

Bed pads are used in hospitals, long term care facilities, and private home to protect bedding, mattresses against any bodily exudates that might deposit on the bed, penetrate and damage the mattress. Bed pads have been developed and placed on top of the bed directly beneath the patient disposed on the bed. The bed pad can consist of an absorbent pad comprising a liquid permeable topsheet, a liquid impermeable backsheet and an absorbent layer disposed between the topsheet and the backsheet.

U.S. Patent application 4,097,943 discloses an absorbent pad suitable for use over hospital bedding beneath incontinent patients.

A bed pad may comprise in addition to a center pad comprising an absorbent layer, an additional layer of material which is three times as long as the center pad. The center pad is disposed at the center of the additional layer such that the bed pad comprises a first and second wing. Alternatively, the first and second wing may be made of two separate layers and may be directly attached to the center pad. The bed pad can be secured to the bed or mattress via the first and second wing.

U. K. Patent application GB 2501561 A discloses a bed pad having opposing wings of material extending beyond edges of the absorbent pad to tuck under a mattress in order to secure the bed pad to the mattress.

In both bed pad embodiments, when the bed pad has been soiled, the bed pad can be replaced and disposed of when required without the necessity of changing and laundering textile sheets of the bed. However, when the bed pad comprises one or more wings, the bed pad is discarded with the one or more wings.

It will be beneficial to provide a more sustainable bed pad.

It will be also advantageous to provide a bed pad wherein one can place the adequate absorbent pad according to the usage conditions of the bed pad. There is thus further a need to provide a more flexible bed pad providing different types of absorbent layers, or different types of wings in order to accommodate to more diverse usage conditions or to fit different sizes of beds or other surfaces to be protected from any liquid bodily exudate contaminations.

### SUMMARY OF THE INVENTION

A bed pad is provided and comprises an absorbent pad having a first and second lateral edge. The absorbent pad comprises a liquid permeable topsheet, a liquid impermeable backsheet and an absorbent layer located between the topsheet and the backsheet. The bed pad further comprises at least one wing. The one or more wings have a first and a second lateral edge. One or more fasteners are disposed at or adjacent to each of the first and second lateral edges of the absorbent pad and/or at or adjacent to one or both of the first and second lateral edges of the one or more wings, such that the one or more wings are able to be releasably engaged with the absorbent pad at or adjacent to the first and/or second lateral edge of the absorbent pad.

A package is provided and comprises:
- One or more absorbent pads, each having a first and second lateral edge and comprising a liquid permeable topsheet, a liquid impermeable backsheet and an absorbent layer located between the topsheet and the backsheet,
- One or more wings, each having a first and a second lateral edge,
- One or more fasteners disposed at or adjacent to each of the first and second lateral edges of the absorbent pad and/or at or adjacent to one or both of the first and second lateral edges of the one or more wings.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
Fig. 1 is a plan view of a bed pad with a portion of a topsheet and a wing cut away to show an absorbent layer and a backsheet, both of which underlie the topsheet cut-away;
Fig. 2 is a schematic sectional view taken along the line 2-2 of Fig. 1;
Fig. 3 is a perspective view of the bed pad of Fig. 1 placed on the bed and secured to the mattress;
Fig. 4 is a schematic sectional view of Fig. 3 taken along the line 4-4 of Fig. 3;
Fig. 5 is a schematic sectional view according to one non-limiting embodiment of the present invention;
Fig. 6 is a perspective view of a bed pad with only one wing according to one non-limiting embodiment of the present invention;
Fig. 7 is a perspective view of the bed pad of Fig. 6 placed on the bed and secured to the mattress;
Fig. 8 is a schematic sectional view of Fig. 7 taken along the line 8-8 of Fig. 7;
Fig. 9 is a schematic sectional view according to one non-limiting embodiment of the present invention;
Fig. 10 is a schematic sectional view according to one non-limiting embodiment of the present invention;
Fig. 11 is a plan view of a wing having on the first lateral edge a stripe of hook fasteners;
Fig. 12 is a zoomed view of a portion of the stripe of hook fasteners of Fig. 11;
Fig. 13 is fragmentary perspective view, partially broken away, of a first lateral edge of a wing to which a tape tab has been applied;
Fig. 14A-14D are plan views of a wing illustrating suitable fasteners configurations.

### DETAILED DESCRIPTION OF THE INVENTION

### Definition of terms

The term "bed pad" as used herein refers to a disposable product for an adult incontinent person but also for a baby and which is placed beneath the body of the wearer to absorb and contain the various liquid bodily exudates discharged from the body. A bed pad comprises a disposable absorbent pad which has a topsheet, a backsheet, an absorbent layer and optionally one or more wings, with the absorbent layer normally placed between the topsheet and the backsheet. The one or more wings may be washable or may be disposable. The bed pad can be put over a mattress but also over any types of surface to be protected against any liquid bodily exudates.

The term "disposable" as used herein means when referring to an absorbent pad that the absorbent pad is intended to be discarded after a single use. When referring to a wing, the term "disposable" means that the wing can be used once or several times (such as up to 10 times) before being discarded.

The term "absorbent layer" as used herein refers to a component, which is placed or is intended to be placed within a bed pad and which comprises an absorbent material which may be enclosed in a core wrap. The absorbent layer is typically the component of an absorbent pad of the bed pad which comprises all, or at least the majority of an absorbent material, preferably fibrous in nature and/or with a superabsorbent material and has the highest absorbent capacity of all the components of the bed pad.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essential of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "advantageously" and the likes also qualify features which are not intended to limit the scope of the claims unless specifically indicated to do so.

The term "cellulosic fiber" as used herein refers to natural fibers which typically are wood pulp fibers. Applicable wood pulps include chemical pulps, such as Kraft, sulfite, and sulfate pulps, as well as mechanical pulps including, for example, groundwood, thermomechanical pulp and chemically modified thermomechanical pulp. Pulps derived from both deciduous trees (hereinafter, also referred to as "hardwood") and coniferous trees (hereinafter, also referred to as "softwood") may be utilized. The hardwood and softwood fibers can be blended, or alternatively, can be deposited in layers to provide a stratified web.

The term "fastener" refers to a device permanently attached at a first layer which is able to releasably engage a second layer either in a mechanical or chemical way.

The term "adjacent to a first and/or second lateral edge of a wing" as used herein means that one or more fasteners are disposed within an area spaced inboard from a first and/or second lateral edge of the wing. The area has a width which is from 1 to 30 % of a width of the wing. If the one or more fasteners are provided on a tape tab, the one or more fasteners are disposed laterally outboard beyond the first and/or second lateral edge of the wing.

The term "adjacent to a first and/or second lateral edge of an absorbent pad" as used herein means that one or more fasteners are disposed within an area spaced inboard from a first and/or second lateral edge of the absorbent pad. The area has a width which is from 1 to 30 % of a width of the absorbent pad. If the one or more fasteners are provided on a tape tab, the one or more fasteners are disposed laterally outboard beyond the first and/or second lateral edge of the absorbent pad.

The term "nonwoven web" as used herein refers to a manufactured material, web, sheet or batt of directionally or randomly oriented fibers, bonded by friction, and/or cohesion and/or adhesion, excluding paper and products which are woven, knitted, tufted, stitch-bonded, incorporating binding yarns or filaments, or felted by wet milling, whether or not additionally needled. The fibers may be of natural or man-made origin. The fibers may be staple or continuous filaments or be formed in situ. The porous, fibrous structure of a nonwoven may be configured to be liquid permeable or impermeable, as desired.

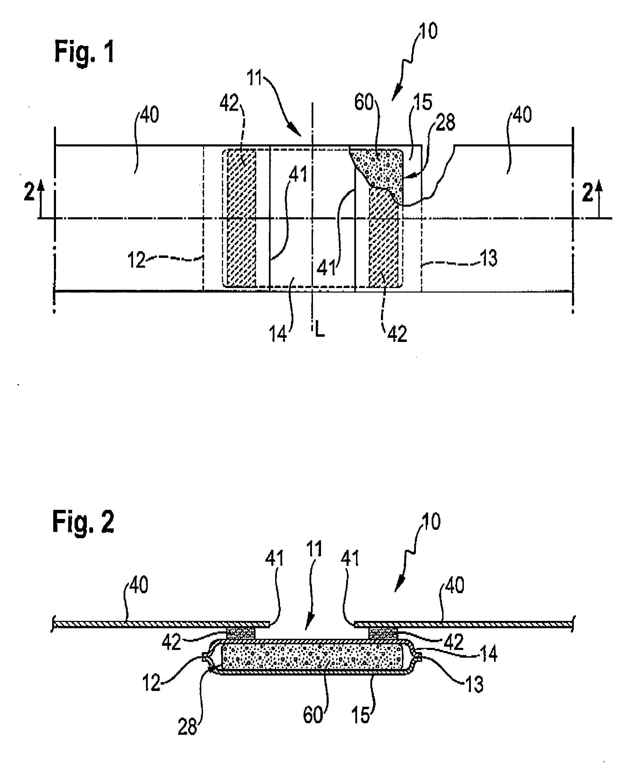

Fig. 1 and 2 show a bed pad 10 which comprises an absorbent pad 11. The absorbent pad 11 has a longitudinal central axis L and a transverse central axis T. The longitudinal axis L of the absorbent pad 11 is the imaginary line separating the absorbent pad 11 along its length in two halves. The transverse central axis T of the absorbent pad 11 is the imagery line perpendicular to the longitudinal axis L in the plane of the absorbent pad 11 and going through the middle of the length of the absorbent pad 11. The absorbent pad 11 has a first and second lateral edge (12, 13). The first and second lateral edge (12, 13) of the absorbent pad 11 are the lateral edges of an absorbent pad 11 running generally parallel to the longitudinal axis L of the absorbent pad 11. The absorbent pad 11 comprises a liquid permeable topsheet 14, a liquid impermeable backsheet 15 and an absorbent layer 28. The absorbent layer 28 is located between the topsheet 14 and the backsheet 15.

The bed pad 10 may have any suitable length and width required for an adult incontinent person or for a baby. Hence, generally the bed pad 10 to be disposed beneath an adult incontinent person or a baby will have a width from 300 mm to 1500 mm or from 500 mm to 1000 mm and a length from 200 mm to 2000 mm or from 500 mm to 1500 mm.

When a bed pad 10 is placed beneath an adult incontinent person in hospitals, the mattress of the bed has to be sufficiently protected from any liquid bodily exudate contaminations.

The bed pad 10 is also useful to assist the caregiver for toilet-training his child when it is desired to not use diapers any more. The bed pad 10 may thus be used in that case to cover the mattress for temporary protection. Alternatively, one may consider using the bed pad 10 as a device to isolate any suitable surface, such as a table on which the baby is changed by the caregivers. The suitable surface may also include, but not limited to, bed sheets, mattresses, bet mats, diaper changing tables, sofas, chairs, seats and floors.

The liquid permeable topsheet 14 may include a woven fabric, a nonwoven web, a polymer film, a film-nonwoven fabric laminate or the like, as well as combinations thereof. A nonwoven web useable as topsheet 14 or comprised by a topsheet 14 may include, for example, an airlaid nonwoven web, a spunbond nonwoven web, a meltblown nonwoven web, a bonded-carded web, a hydroentangled nonwoven web, a spunlace web or the like, as well as combinations thereof. The topsheet 14 may be made of a nonwoven web having a basis weight from 5 to 50 gsm or from 10 to 20 gsm.

Other conventional examples of suitable materials for constructing the topsheet 14 may include rayon, bonded- carded webs of polyester, polypropylene, polyethylene, nylon, or other heat-bondable fibers, finely perforated film webs, net-like materials, and the like, as well as combinations thereof. When the topsheet 14 is a film or a film laminate, the film should be fluid permeable. The topsheet 14 may e.g. be rendered fluid permeable by aperturing. Alternatively, the topsheet 14 may be made of a composite material comprising a polymer and a nonwoven fabric material.

As shown in Fig. 1, the topsheet 14 extends beyond the absorbent layer 28. The topsheet 14 may be bonded to the backsheet 15, by any conventional means such as adhesives, heat/pressure sealing, ultrasonic bonding, or any other means or methods as are known in the art. It is contemplated, however, that the topsheet 14 can have the same extent as the absorbent layer 28 and/or backsheet 15 or can have an extent less that the absorbent layer 28 and/or backsheet 15.

The liquid impermeable backsheet 15 may be a polymeric film, a woven fabric, a nonwoven web or the like, as well as combinations or composites thereof. For example, the liquid impermeable backsheet 15 may include a polymer film laminated to a woven or nonwoven web. The polymer film can be composed of polyethylene, polypropylene, polyester, silicone or the like, as well as combinations thereof. For example, the backsheet 15 may be spunbond/meltblown/spunbond nonwoven treated with a polyethylene coating. Additionally, the backsheet 15 may be embossed, have a printed design, have a printed message to the consumer, and/or may be at least partially colored. In case that the backsheet 15 is made of a permeable material such as a nonwoven web, the absorbent pad 11 may include a barrier layer disposed between the absorbent layer 28 and the nonwoven backsheet.

The backsheet 15 and/or the topsheet 14 may be attached to the absorbent layer 28 in order to keep the absorbent layer 28 in place between the topsheet 14 and backsheet 15 when the incontinent person is moving on the bed pad 10.

The absorbent layer 28 is configured to absorb the liquid bodily exudates including, but not limited to, urine that passes through the topsheet 14. Suitable absorbent materials 60 which can be used to form the absorbent layer 28 include those absorbent materials 60 conventionally used in an absorbent pad and include absorbent materials, such as, for example, superabsorbent material, cellulose fibers and/or synthetic fibers. Synthetic fibers may be meltblown polymers such as polyester or polypropylene. Another suitable absorbent material is coform which is a meltblown air-formed combination of meltblown polymers, such as polypropylene, and cellulosic fibers.

The absorbent layer 28 may include a superabsorbent material 60, in addition to or in place of the cellulosic fibers. The superabsorbent material 60 may increase the ability of the absorbent layer 28 to absorb a large amount of fluid in relation to its own weight. Generally stated, the superabsorbent material 60 may be a water-swellable, generally water- insoluble, hydrogel - forming polymeric absorbent material, which is capable of absorbing at least about 15, suitably about 30, and possibly about 60 times or more its weight in physiological saline (e.g., saline with 0.9 wt% NaCl).

The superabsorbent material 60 may be formed from organic hydrogel - forming polymeric material, which may include natural material such as agar, pectin, and guar gum; modified natural materials such as carboxymethyl cellulose, carboxyethyl cellulose, and hydroxypropyl cellulose; and synthetic hydrogel- forming polymers. Synthetic hydrogel - forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like.

Other suitable superabsorbent material 60 may include hydrolyzed acrylonitrile grafted starch, acrylic acid grafted starch, and isobutylene maleic anhydride copolymers and mixtures thereof.

The absorbent layer 28 may comprise from 10 to 200 gsm or from 20 to 100 gsm of absorbent material 60.

The bed pad 10 comprises at least one wing 40. The one or more wings 40 have a first and second lateral edge (41, 43). The one or more wings 40 have a longitudinal axis Lw and a transverse axis Tw. The longitudinal axis Lw of the one or more wings 40 is the imaginary line separating the one or more wings 40 along their length in two halves. The transverse central axis Tw of the one or more wings 40 is the imagery line perpendicular to the longitudinal axis Lw in the plane of the one or more wings 40 and going through the middle of the length of the one or more wings 40. The first and second lateral edge (41, 43) of the one or more wings 40 are the edges running generally parallel to the longitudinal axis Lw. In the bed pad 10, the longitudinal axis L of the absorbent pad 11 is substantially parallel to the longitudinal axis Lw of the one or more wings 40. The one or more wings 40 may include a woven fabric, a nonwoven web, a polymer film, a film-nonwoven fabric laminate or the like, as well as combinations thereof.

The one or more wings 40 may be made of a nonwoven web. Alternatively, the one or more wings 40 may be formed from a textile material having water penetration resistance, but still providing good heat transfer characteristics and launderability. Suitable textile materials may be woven fabrics formed of cotton and/or synthetic fibers. Hence, the one or more wings 40 may be made of a breathable material in order to prevent the person laid down on the bed pad sweating. The one or more wings 40 may have a basis weight from 2 to 70 gsm or from 10 to 30 gsm.

One or more fasteners (42, 44, 45) are disposed at or adjacent to each of the first and second lateral edges (12, 13) of the absorbent pad 11 and/or at or adjacent to one or both of the first and second lateral edges (41, 43) of the one or more wings 40.

Hence, the one or more wings 40 are able to be releasably engaged with the absorbent pad 11 at or adjacent to the first and/or second lateral edge (12, 13) of the absorbent pad 11.

The bed pad 10 may comprise a first and a second wing 40, as shown in Fig. 1 and Fig. 2. One or more fasteners 42 may be disposed at or adjacent to the first lateral edge 41 of the first wing 40. The first wing 40 may be thus able to be releasably engaged with the absorbent pad 11 at or adjacent to the first lateral edge 12 of the absorbent pad 11. One or more fasteners 42 may be disposed at or adjacent to the first lateral edge 41 of the second wing 40. The second wing 40 may thus be able to be releasably engaged with the absorbent pad 11 at or adjacent to the second lateral edge 13 of the absorbent pad 11.

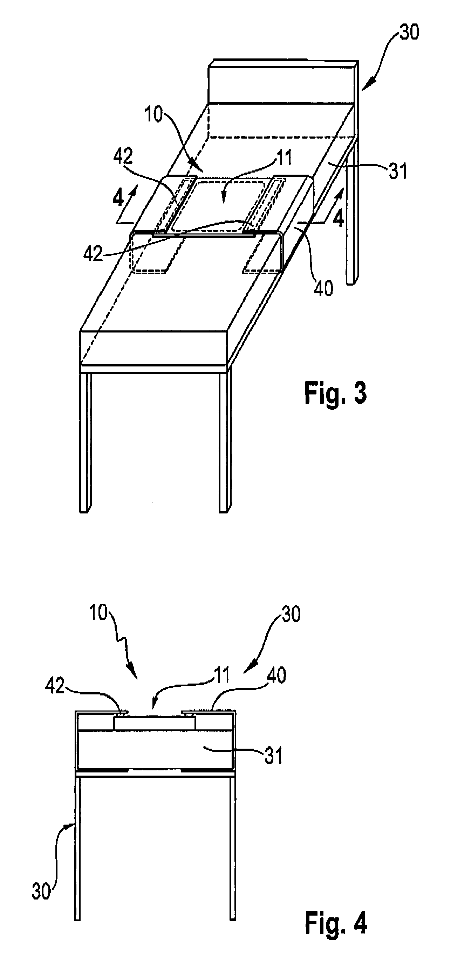

As illustrated in Fig. 3 and Fig. 4, a bed 30 comprises a mattress 31 and the bed pad 10. The first and second wing 40 of the bed pad 10 which is releasably engaged with the absorbent pad 11 at or adjacent to the respective first and second lateral edge (12, 13) of the absorbent pad 11 have been tucked under the mattress 31. The first and second wing 40 of the bed pad 10 allow securing the bed pad 10 to the mattress. Additionally, as the first and second wing of the bed pad 10 can be detached from the absorbent pad 11, the soiled absorbent pad 11 can be readily disposed. The first and second wing 40 can then be reused several times without a need to be laundered in-between. The first and second wing 40 can remain tucked under the mattress 31 when changing the soiled absorbent pad 11, which provides a more flexible bed pad 10. The first and second wing 40 may be laundered when the first and second wing 40 are made of appropriate material.

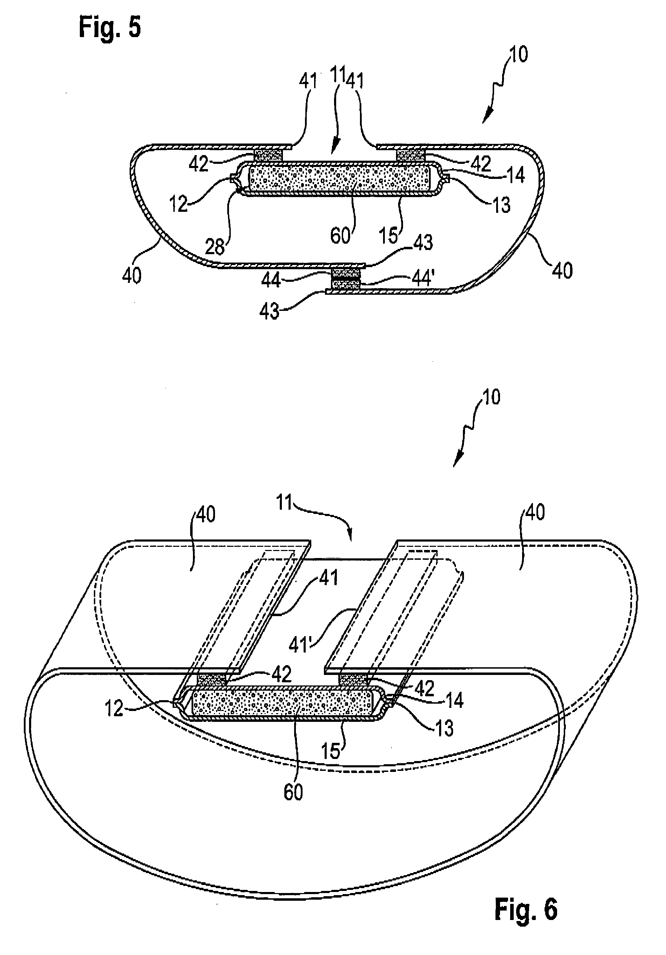

One or more fasteners 44 which are disposed at or adjacent to the second lateral edge 43 of the first wing 40 may be able to be releasably engaged with one or more fasteners 44' disposed at or adjacent to the second lateral edge of the second wing 40, as shown in Fig. 5. Prior to or after positioning the first and second wing 40 under the mattress 31, the first and second wing 40 can be releasably attached to each other at their respective second lateral edges 43. The first lateral edges 41 of each of the first and second wing 40 are then engaged with the respective first and second lateral edge (12, 13) of the absorbent pad 11. Once in place, the bed pad 10 may be relatively tightly secured around the mattress 31 of the bed 30. This optional feature may be of interest in case when the person laid down on the bed may move and displace the bed pad 10 with the first and second wing 40.

Alternatively, the bed pad 10 may comprise only one wing 40, as shown in Fig. 6. One or more fasteners 42 disposed at or adjacent to the first lateral edge 41 of the only one wing may 40 be able to be releasably engaged with the absorbent pad 11 at or adjacent to the first lateral edge 12 of the absorbent pad 11. One or more fasteners 42 disposed at or adjacent to the second lateral edge 41' of the only one wing 40 may be able to be releasably engaged with the absorbent pad 11 at or adjacent to the second lateral edge 13 of the absorbent pad 11.

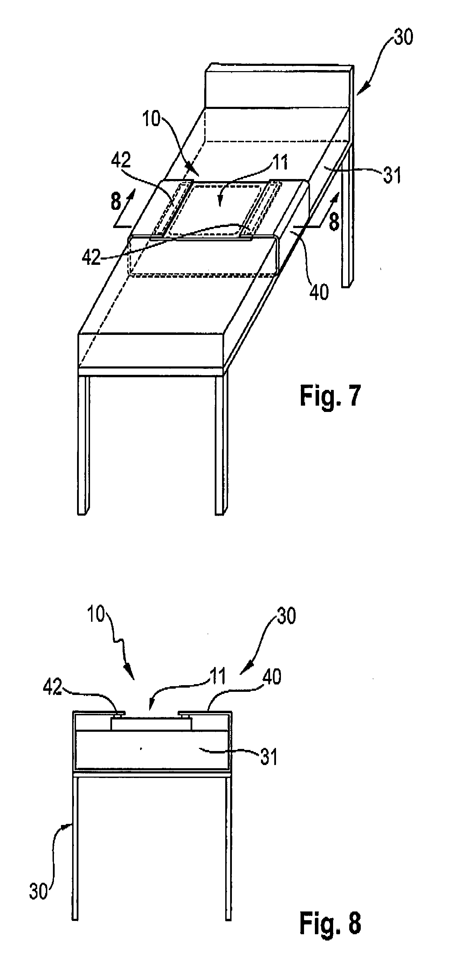

As illustrated in Fig. 7 and Fig. 8, the bed 30 comprises the bed pad 10 having only one wing 40. The only one wing 40 of the bed pad 10 which is releasably engaged with the absorbent pad 11 at or adjacent to the respective first and second lateral edge (12, 13) of the absorbent pad 11 is supplemented below the mattress 31. The bed pad 10 surrounds the mattress 31. Hence, the only one wing 10 of the bed pad 10 increases the stability of the bed pad 10 to the mattress 31 in case the incontinent person is moving on the mattress 31 overnight. The absorbent pad 11 can also be changed without removing the only one wing from the bed. Such bed pad 10 may be also suitable to secure any types of surface like a diaper changing table.

Additionally, the absorbent pad 11 may comprise one or more fasteners 45 which are disposed at or adjacent to each of the first and second lateral edges (12, 13) of the absorbent pad 11. The one or more fasteners 45 at each of the first and second lateral edges (12, 13) of the absorbent pad 11 may be provided either on the topsheet 14 or the backsheet 15 or between the topsheet 14 and backsheet 15.

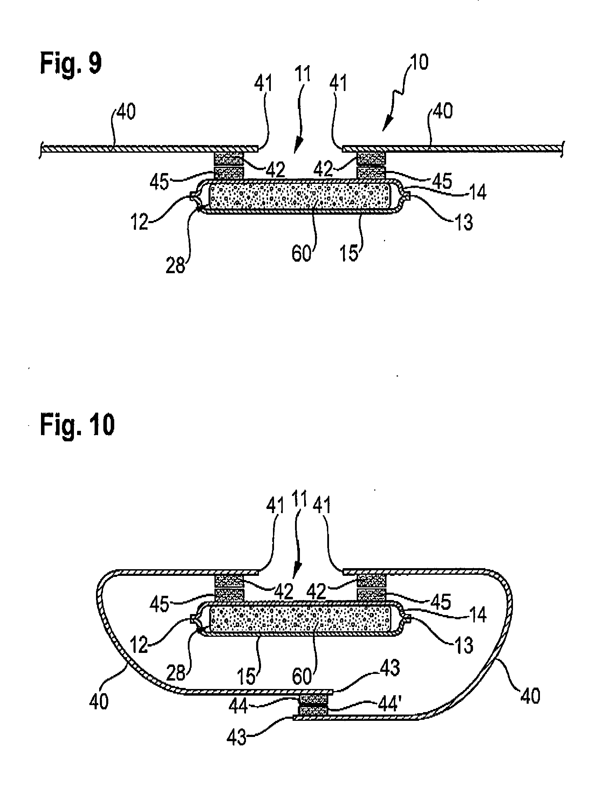

When the bed pad 10 comprises a first and second wing 40, the first wing 40 may be able to be releasably engaged with the absorbent pad 11 at or adjacent to the first lateral edge 12 of the absorbent pad 11. The second wing 40 may be able to be releasably engaged with the absorbent pad 11 at or adjacent to the second lateral edge 13 of the absorbent pad 11, as shown in Fig. 9 or another example in Fig. 10.

The only one wing 40 may be also able to be releasably engaged with the absorbent pad 11 at or adjacent the first and second lateral edge (12, 13) of the absorbent pad 11.

When the absorbent pad 11 further comprises one or more fasteners 45 which are disposed at or adjacent to each of the first and second lateral edges (12, 13) of the absorbent pad 11, the one or more fasteners 45 on the absorbent pad 11 provides a complementary fastener to the one or more fasteners 42 of the first and second wing 40, or the only one wing 40 (such as a hook fastener and a complimentary loop fastener). Hence, the reliable fastening of the absorbent pad 11 with the first and second wing 40 or the only one wing 40 is optimized. The bed pad 10 may be firmly secured to the mattress 31.

One or more fasteners 45 of any of the first and second lateral edge (12, 13) of the absorbent pad 11 may be complementary to one or more fasteners 42 which are disposed at or adjacent to the first or second lateral edge 41 of the one or more wings. When the one or more fasteners 45 on the absorbent pad 11 are complementary to the one or more fasteners 42 of the one or more wings 40, the strength of the releasable attachment is enhanced. It is unlikely that the one or more wings 40 are released without the intervention of a caregiver.

One or more fasteners (42,45) may be mechanical or chemical fasteners. The mechanical fastener may be a hook fastener or an interlocking fastener (such as a button which can engage with a complimentary button hole). A hook fastener may be releasably engageable with e.g. a nonwoven web or with a complimentary loop fastener (such as a knitted or nonwoven web).

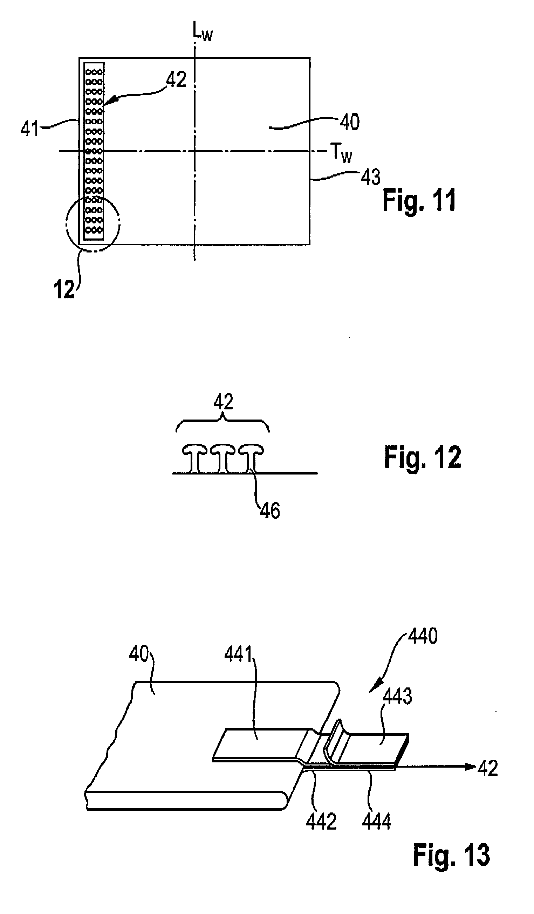

One or more fasteners (42, 45) may be a hook or a loop fastener. As depicted in Fig. 11 and 12, a hook fastener 42 may comprise a plurality of resiliently flexible spaced hook members 46.

One or more loop fasteners 45 may be disposed at or adjacent to each of the first and second lateral edges (12, 13) of the absorbent pad 11. When one or more hook fasteners 42 are disposed at or adjacent to the first lateral edge 41 of the first and second wing 40, the first and second wing 40 may be releasably engaged with the absorbent pad 11. Indeed, the hook fasteners 42 of the first and second wing 40 may engage with the loop fasteners 45 of the absorbent pad 11.

The loop fastener may be the fibrous loops of a nonwoven web constituting the topsheet 14 of the absorbent pad 11. Alternatively, the loop fastener may be provided by a knitted fabric.

The mechanical fasteners may be interlocking fasteners. An interlocking fastener may comprise a tap and a slot member. For instance, a tap member may be disposed at or adjacent the first lateral edge of one or more wings 40. The slot member may be disposed at the first and second lateral edge (12. 13) of the absorbent pad 11. The slot member is a portion of the interlocking fastener through which the tab member of the one or more wings 40 is passed in order to engage of fasten the one or more wings 40 to the absorbent pad 11.

A chemical fastener may be pressure sensitive adhesive. Pressure-sensitive adhesives are typically formulated to adhere to a surface at ambient temperature by briefly applying light pressure alone. Pressure-sensitive adhesives may be emulsion, solvent, and hot melt pressure-sensitive adhesives. Emulsion pressure-sensitive adhesives may include a wide variety of polymeric materials (usually thermoplastic or elastomeric) dispersed in a continuous aqueous phase. The solvent pressure-sensitive adhesives may consist of thermoplastic or elastomeric compositions dissolved in an appropriate aqueous or organic solvent at levels from 1% to 99% solids. All polymer systems in this class are in solutions in the solvent and are not to be confused with emulsions, where the polymer is actually in dispersions. Hot melt pressure-sensitive adhesives may consist of compositions that are capable of being melted and applied to a substrate in its molten state which then cool and solidify relatively quickly. Examples of suitable hot melt adhesives are ethylene-vinyl acetate (EVA) or rubber-based hot melt pressure-sensitive adhesives.

A separate release liner may be provided to cover the pressure-sensitive adhesive. The release liner may be polyethylene or paper or any suitable equivalent thereof that will provide protection. The release liner may be treated on its surface which contacts the fastener so that it has moderate adhesive attachment to the fastener. For instance, a silicone treatment has been found to provide this moderate adhesive attachment with pressure sensitive adhesives.

One or more fasteners 45 may be disposed at or adjacent to each of the first and second lateral edges (12, 13) of the absorbent pad 11 on the topsheet 14 or the backsheet 15. One or more fasteners 42 may be disposed at or adjacent to each of the first lateral edge 41 of the wing 40. The one or more fasteners 42 may be disposed outboard of each of the first and second lateral edges (41, 43) of the wing 40. For instance, the one or more fasteners 42 may be disposed on a fastening portion 444 of a tape tab 440 as shown in Fig. 13.

A fastener (42, 45) may be provided on a tape tab. The tape tab may be permanently attached to the first and/or second lateral edge (12, 13) of the absorbent pad 11, e.g. on the topsheet 14 or backsheet 15. A portion of the tape tab may extend laterally outboard beyond the first and/or second lateral edge (12, 13) of the absorbent pad 11. The fastener, such as a hook fastener or a pressure sensitive adhesive may be provided on the portion of the tape tab extending outboard the first and/or second lateral edge (12, 13) on the absorbent pad 11.

The tape tab may be permanently attached to the first lateral edge 41 of the one or more wings 40. A portion of the tape tab may extend laterally outboard beyond the first lateral edge 41 of the one or more wings 40. The fastener, such as a hook fastener or a pressure sensitive adhesive may be provided on the portion of the tape tab extending outboard the first lateral edge 41 of the one or more wings 40.

Referring now to Fig. 13, an example of a tape tab 440 is depicted. A tape tab may 440 have at least three elements, which are a front surface portion 441, a back surface portion 442, and the fastening portion 444. The tape tab may be attached to the first lateral edge 41 of the wing 40 by the front and back surface portions (441, 442), but it does not need to be. The front surface portion 441 is that portion of the tape tab which is attached to a front surface of the one or more wings 40 with an attachment mean 445. The back surface portion 442 is that portion of the tape tab which is attached to the back surface of the one or more wings 40 with an attachment mean 445. The fastening portion 444 is the portion of the tape tab 42 outboard the first and/or second lateral edge (41,43) of the wing 40. The fastening portion 444 may comprise either a mechanical fastener, e.g. a patch of hook fasteners or a chemical fastener such as a pressure-sensitive adhesive which is able to be releasably engaged to one of the first or second lateral edge (12, 13) of the absorbent pad 11. The tape tab may be provided with a separate release line 443.

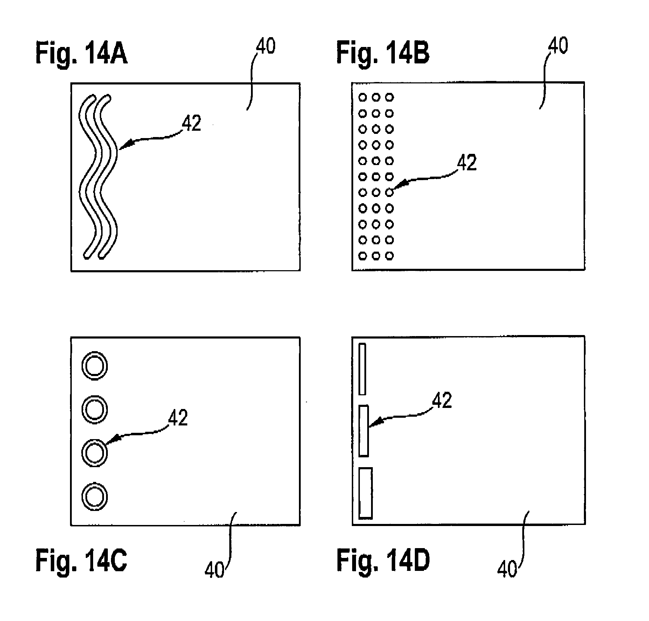

One or more fasteners may have a pattern selected from the group consisting of stripes, wavy pattern, dots, circles and combinations thereof. Various suitable pattern configurations for the one or more fasteners can be contemplated as illustrated in Fig. 14A-14D. The pattern of one or more fasteners may be used as a targeting indicia component in order to indicate, facilitate and/or compel correct positioning and engagement of the one or more wings 40 with the absorbent pad 11.

The one or more fasteners 42 may be continuous or discontinuous stripes at the first and/or second lateral edge (12, 13) of the absorbent pad 11 and/or the one or more wings 40.

The one or more wings 40 may comprise a continuous stripe of hook fasteners or continuous strip of pressure-sensitive adhesive disposed at or adjacent the first and/or second lateral edge (41, 43) of the one or more wings 40.

A package is provided and comprises:
- One or more absorbent pads 11, each having a first and second lateral edge (12, 13) and comprising a liquid permeable topsheet 14, a liquid impermeable backsheet 15 and an absorbent layer 28 located between the topsheet 14 and the backsheet 15,
- One or more wings 40, each having a first and a second lateral edge (41, 43),
- One or more fasteners (42, 44, 45) disposing at or adjacent to each of the first and second lateral edges (12, 13) of the absorbent pad 11 and/or at or adjacent to one or both of the first and second lateral edges (41,43) of the one or more wings 40.

The package may comprise a ratio between a wing 40 and an absorbent pad 11 of at least 1:2, such as 1:2 to 1:20 or 1:2 to 1:10. For instance, the package may comprise two wings 40 and ten absorbent pads 11.

The package may comprises a first type of absorbent pad having a first type of absorbent layer and a second type of absorbent pad having a second type of absorbent layer. The first type of absorbent layer differs from the second type of absorbent layer. The first and second absorbent layer may have different amount of absorbent material such that the first type and second type of absorbent layer may have different absorption capacities. Hence, the package may comprise different types of absorbent pad for specific usage conditions. In hospitals, some adult incontinent persons may need a bed pad comprising an absorbent pad having a relatively high amount of absorbent material, especially when these adult incontinent persons are subjected to urinate more frequently than a person in a good health. Different types of absorbent pad having different absorption capacities may be useful when it is required to place a bed pad beneath an adult incontinent person or baby for a long time. This is the case in hospitals when the bed cannot be changed as often as desirable because there are not enough staff members available.

Different types of bed pad may also be useful for toddlers on toilet-training program. Hence, the consumer with a single package is able to use the appropriate absorbent pads at the appropriate time. The package of the present invention avoids the consumer purchasing a package of each type of absorbent pad. The package of the present invention does also not require excess storage space.

The ratio of the different types of absorbent pads within the single package may be any suitable ratio. For example, the first type and the second type of absorbent pad may be present in a ratio between 12:1 and 3:1. The first type of absorbent pad may have a first type of absorbent layer with a higher amount of absorbent material than the second type of absorbent layer of the second type of absorbent pad. The first type of absorbent pad may be thus used in a bed pad to be place beneath a person for a long time, e.g. overnight.

The package may comprise a plurality of absorbent pads. Each absorbent pad may have a size. The size of each absorbent pad may differ from each other. The package may comprise a plurality of wings. Each wing may have a size. The size of each wing may differ from each other.

The package can thus provide flexibility for the caregiver to place the right size of the absorbent pad with the right size of the one or more wings according to the size of the bed, or size of the wearer (from an adult or a baby), or size of the surface to be isolated from any contaminations with the liquid bodily exudates.

### Example:

The following bed pad according to the invention was prepared: An absorbent pad having as a dimension 75mm x 65 mm was prepared with a 15 gsm nonwoven topsheet having a dimension of 75mm x 65 mm, a 18 gsm polyethylene backsheet having a dimension of 75mm x 65 mm and an absorbent layer comprising 60 gsm cellulosic fibers. The absorbent layer has a dimension of 60 mm x 60 mm. A first and second wing each having a dimension of 65 mm x 45 mm and made of a 25 gsm nonwoven web comprises at each first lateral edge a continuous stripe of hook fasteners.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. Bed pad comprising:
- an absorbent pad having a first and second lateral edge,
wherein the absorbent pad comprises a liquid permeable topsheet, a liquid impermeable backsheet and an absorbent layer located between the topsheet and the backsheet,
- at least one wing, wherein the one or more wings have a first and a second lateral edge, **characterized in that** one or more fasteners are disposed at or adjacent to each of the first and second lateral edges of the absorbent pad and/or at or adjacent to one or both of the first and second lateral edges of the one or more wings,
such that the one or more wings are able to be releasably engaged with the absorbent pad at or adjacent to the first and/or second lateral edge of the absorbent pad.

2. Bed pad according to claim 1 comprising a first and second wing, wherein one or more fasteners disposed at or adjacent to the first lateral edge of the first wing are able to be releasably engaged with the absorbent pad at or adjacent to the first lateral edge of the absorbent pad, and wherein one or more fasteners disposed at or adjacent to the first lateral edge of the second wing are able to be releasably engaged with the absorbent pad at or adjacent to the second lateral edge of the absorbent pad.

3. Bed pad according to claim 2 wherein one or more fasteners disposed at or adjacent to the second lateral edge of the first wing are able to be releasably engaged with one or more fasteners disposed at or adjacent to the second lateral edge of the second wing.

4. Bed pad according to claims 2 and 3 wherein one or more fasteners are disposed at or adjacent to each of the first and second lateral edges of the absorbent pad;
such that the first wing is able to be releasably engaged with the absorbent pad at or adjacent to the first lateral edge of the absorbent pad; and
the second wing is able to be releasably engaged with the absorbent pad at or adjacent to the second lateral edge of the absorbent pad.

5. Bed pad according to claim 1 comprising only one wing wherein one or more fasteners disposed at or adjacent to the first lateral edge of the only one wing is able to be releasably engaged with the absorbent pad at or adjacent to the first lateral edge of the absorbent pad, and wherein one or more fasteners disposed at or adjacent to the second lateral edge of the only one wing is able to be releasably engaged with the absorbent pad at or adjacent to the second lateral edge of the absorbent pad.

6. Bed pad according to claim 5 wherein one or more fasteners are disposed at or adjacent to each of the first and second lateral edges of the absorbent pad;
such that the only one wing is able to be releasably engaged with the absorbent pad at or adjacent to the first and second lateral edge of the absorbent pad.

7. Bed pad according to claims 4 and 6 wherein one or more fasteners on any of the first and second lateral edge of the absorbent pad correspond to one or more fasteners which are disposed at or adjacent to the first or second lateral edge of the one or more wings.

8. Bed pad according to any preceding claims wherein the one or more fasteners are mechanical or chemical fasteners.

9. Bed pad according to claim 8 wherein the mechanical fasteners are selected from the group consisting of hook fastener, loop fastener, interlocking fastener and combinations thereof, or wherein the chemical fasteners are pressure sensitive adhesives.

10. Bed pad according to any of the preceding claims wherein one or more fasteners are provided on a tape tab.

11. Bed pad according to any of the preceding claims wherein one or more fasteners have a pattern selected from the group consisting of stripes, wavy pattern, dots, circles and combinations thereof.

12. Bed pad according to any the preceding claims wherein the one or more wings comprises a continuous stripe of hook fasteners disposed at or adjacent the first and/or second lateral edge of the one or more wings.

13. A package comprising:
- One or more absorbent pads, each having a first and second lateral edge and comprising a liquid permeable topsheet, a liquid impermeable backsheet and an absorbent layer located between the topsheet and the backsheet,
- One or more wings, each having a first and a second lateral edge,
- One or more fasteners disposed at or adjacent to each of the first and second lateral edges of the absorbent pad and/or at or adjacent to one or both of the first and second lateral edges of the one or more wings.

14. The package according to any of claim 13 wherein the package comprises a first type of absorbent pad having a first type of absorbent layer and a second type of absorbent pad having a second type of absorbent layer and wherein the first type of absorbent layer differs from the second type of absorbent layer.

15. The package according to any of claim 13 wherein the package comprises a plurality of absorbent pads, each absorbent pad having a size, wherein the size of each absorbent pad differs from each other and/or wherein the package comprises a plurality of wings, each wing having a size, wherein the size of each wing differs from each other.
